# EUROPEAN PATENT APPLICATION

(11) **EP 4 310 168 A1**
(43) Date of publication of application: **24.01.2024**
(21) Application number: 22186207.1
(22) Date of filing: 21.07.2022
(51) Int. Cl.: C12M 1/00, C12M 3/00

(54) **PHOTOBIOREACTOR SYSTEM FOR THE CULTIVATION OF PHOTOSYNTHETIC MICRO-ORGANISMS IN ANIMAL HOUSES**

(71) Applicant: Acheron GmbH, 28217 Bremen (DE)
(72) Inventor: MEISSNER, Kai, 28199 Bremen (DE); KAPIESKE, Denis, 28201 Bremen (DE); LANSING, Lucas, 28213 Bremen (DE); GLOCKOW, Till, 28201 Bremen (DE)
(74) Representative: Müller-Boré & Partner Patentanwälte PartG mbB

(57) **Abstract**

The present invention relates to photobioreactor systems for the cultivation of photosynthetic microorganisms, as well as to related methods and uses of said systems for converting waste gases produced in an animal house to biomass, cultivating photosynthetic microorganisms, and/or filtering the air in animal houses.

## Description

The present invention relates to photobioreactor systems for the cultivation of photosynthetic microorganisms, as well as to related methods and uses of said systems for converting waste gases produced in an animal house to biomass, cultivating photosynthetic microorganisms, and/or filtering the air in animal houses.

In terms of food production, global product demand will increase by up to 70% by 2050. At the same time, the available land that can be used for agriculture is already exhausted or can only be expanded through progressive deforestation. Against this background, investments in the development and provision of efficiency-increasing and at the same time resource-saving, sustainable cultivation and breeding methods are of essential importance for humankind. This requires closed-loop systems that are characterized by highly efficient use of cultivation/production area and raw materials employed within a production process.

Urban and indoor farming has been credited in the recent past with a high potential for increasing yields per acre. However, even with this approach, conventional agricultural enterprises rely mainly on monocultures, both in animal and in crop farming. Since the by-products of such pure monocultures, in particular carbon oxide, phosphorus, nitrogen, and sulfur compounds, as well as contaminated wastewater, cannot be re-used directly and transport is uneconomical in terms of their market value, they generally represent waste materials that are either disposed of at high cost or released uncontrolled into the environment as emissions. In this regard, conventional animal fattening poses a particular problem. In Germany alone, more than 200,000 farms are involved in the production of pigs and poultry, resulting not only in enormous emissions of CO, CO₂, ammonia and other nitrogen and sulfur compounds, but also of feces and slurry, which represent a barely tolerable burden on the environment. While feces and manure from animal fattening can be converted into more valuable substances with the help of biofilters or biogas plants, no approaches have yet been described with which the gaseous emissions from animal houses can be reduced and simultaneously used for the production of valuable substances. In particular, there is a lack of approaches for concrete apparatuses that make advantageous use of the given conditions prevailing in conventional animal houses in order to be able to utilize the animal emissions contained in the air for the production of valuable compounds.

Photosynthetic microorganisms (in the following referred to as PMO), such as eukaryotic (micro)algae or cyanobacteria bind carbon dioxide by photosynthesis and are also able to filter nitrogen and sulfur compounds or other gaseous or aerosol-bound substances from the air as biofilters. For their growth, these photosynthetic microorganisms require not only light, carbon dioxide, water, and inorganic nutrients, but also elevated temperatures in particular, which are typically in the physiological range of 25 to 45 °C. Under optimal conditions, a sufficient carbon source and light energy, PMO can double in biomass within 12 hours, converting large amounts of gaseous substances into biogenic valuable substances through photosynthesis.

In PMO cultivation processes, carbon usually accounts for more than half of the dry weight of the biomass produced. However, the cells of PMO are also rich in a wide variety of biogenic and bioactive substances, such as amino acids, proteins, carbohydrates, oligo- and polysaccharides, unsaturated fatty acids, and numerous secondary metabolites, such as vitamins, dyes, or antibiotics. Moreover, nowadays the choice of PMO is not limited to naturally occurring strains, but with the help of genetic engineering methods, recombinant species are also accessible, producing certain biogenic constituents in high yields. This makes high-value products for energy or health applications, such as enzymes or even therapeutic antibodies accessible.

Due to the high importance of PMO for sustainable production processes, a large number of cultivating reactor concepts and applications have been described. For example, there are two main types of large-scale algal biomass production systems: open and closed (photobioreactors). Open systems are classified into natural water bodies (lakes, lagoons, ponds) and artificial ponds or tanks and include shallow large ponds, tanks, circular ponds, raceway ponds, or high-rate ponds. Although open ponds have the advantage of being easier and cheaper to build and operate than most closed systems, they have the disadvantages of often involving poor light utilization by the PMO cells, evaporative losses, diffusion of CO₂ into the atmosphere, and the need for large construction areas. Due to the inefficient stirring mechanisms in open culture systems, mass transfer can also be very poor, resulting in low biomass productivity. In addition, the potential for contamination by viruses, fungi, predators, and other fast-growing microorganisms prohibits the use of such systems in standard animal housing.

There are also different forms of closed photobioreactors, such as airlift reactor, stirred reactor, flat plate reactor or tubular reactor, which can be used for the cultivation of PMO and the production of value-added products. Although many of the disadvantages of open pond reactors are avoided with closed photobioreactors, their main disadvantages are the very high cost of purchase, installation, and operation.

Numerous variations of closed photobioreactor systems are known in the art. For example, US Patent 10,160,941 describes a complex photobioreactor system that can include one or more tubes that can be connected to a fluid to grow or produce microorganisms or biomass such as micro-algae. The system may comprise either a single loop of tubes or an array of tubes. A first fluid may be contained within the tube or tubes, and an inlet port may be provided to introduce a second fluid into the tube or tubes. The tube or tubes may be arranged vertically so that the second fluid rises through the tube or tubes. An outlet port may be provided at the upper end of the tube or tubes to remove the second fluid. The second fluid may be circulated through the system via inlet and outlet tubing, as well as a pump and a replaceable container to hold the second fluid. In a series of tubes, a single inlet and outlet line may be used for each tube.

Despite the numerous variations of closed photobioreactor systems described above, no concrete apparatuses are known so far with which the given conditions in conventional animal houses can be used advantageously in order to be able to utilize the animal emissions contained in the air for the production of valuable substances.

Similarly, systems are known in the art that are based on closed photobioreactors that serve to purify the air. For example, patent application WO 2020/011468 A1 describes an air purification system consisting of a housing containing a bioreactor through which an air stream is blown, and which contains at least one life form capable of at least partially converting the CO₂ contained in the air stream to O₂ by means of photosynthesis. This invention is specifically designed for outdoor/indoor coupling, as it can also advantageously save energy by not sacrificing the warmth or coolness of the indoor space when ventilated with the outdoor air. The outdoor air can enter the indoor space without having to open the windows to reduce the CO₂ content in the indoor air.

In another example, patent application US 2020/0360856 A1 describes an air purifying photobioreactor to improve indoor air quality. The bioreactor consists of a base, which houses the mechanical components, and a container, which contains a liquid mixture of water, as well as a photosynthetic microorganism in a nutrient medium. The bioreactor is of small size (about 1.7 L), equipped with an air pump that draws room air through an air filter into the base of the device. The bioreactor blows the air through the liquid mixture. Through photosynthesis, the CO₂ is converted into O₂. The biomass produced by this process is to be disposed of properly at regular intervals.

However, even though numerous systems are known in the art in which closed photobioreactors serve for air purification, no concrete apparatuses are known so far which can be used under the given conditions in conventional animal houses in order to be able to utilize the animal emissions contained in the air for the production of valuable substances.

Suitable apparatuses, which could be used under the given conditions in conventional animal houses to harvest animal emissions (e.g., chemical components contained in the air) and waste heat from the animals for the production of valuable substances, would have to solve the following problems, which have not been addressed so far.

In conventional animal houses, the entire floor area is required for the animals living in the house (pigs, cattle, sheep, poultry, etc.) to allow maximum freedom of movement. Therefore, a suitable photobioreactor must have the smallest possible footprint, ideally none at all. Due to the metabolic activity of the animals, there is a temperature gradient in conventional animal houses, ranging from about 37 °C near the animal stock on the ground to - depending on outdoor conditions and air conditioning - higher or lower temperatures in the upper air space of the house. Likewise, the highest airborne emission concentrations (e.g., CO₂) occur in the lower part of the animal house. Therefore, a suitable photobioreactor must provide the largest possible contact area in the lower area of the animal house and a rapid mixing of the liquid culture phase in order to be able to tap the ideal growth temperatures and CO₂ concentrations. Also, a suitable photobioreactor must be separable from particulate matter and other sources of contamination by suitable filter systems. Importantly, a suitable photobioreactor must be physically easy to integrate into existing animal houses, and have a high physical robustness to resist mechanical damage to the reactor and its operating elements (lighting fixtures, pumps, valves) by physical contact with the animals. Last but not least, a suitable photobioreactor must be scalable in terms of its individual size and culture volume and easily integratable in series and parallel circuits inside animal houses.

Accordingly, the technical problem underlying the present invention is the provision of a system for the cultivation of photosynthetic microorganisms fulfilling the above requirements.

The solution to the above technical problem is achieved by the embodiments characterized in the claims.

In particular, in a first aspect, the present invention relates to a photobioreactor system for the cultivation of photosynthetic microorganisms, comprising
(a) a cultivation vessel comprising at least one tubular element (101) extending helically around a center axis, wherein the diameter (102; 103) of the helix thus formed continuously increases as the tubular element extends along the center axis,
(b) at least one gas inlet (108) at a first end of the tubular element (101) configured to provide a gas to the tubular element (101),
(c) an equalizing reservoir (201) in fluid connection with a second end (109) of the tubular element (101) configured to provide pressure equalization in the cultivation vessel,
(d) at least one fluid return element (202) fluidly connecting the equalizing reservoir (201) and the first end of the tubular element (101), wherein the tubular element (101) at least partially surrounds the fluid return element (202), and
(e) at least one lighting fixture (110) configured to supply both the tubular element (101) and the fluid return element (202) with light energy of a suitable wavelength, wherein the tubular element (101) at least partially surrounds the lighting fixture (110).

In preferred embodiments, when the photobioreactor system of the present invention is in its position of use, the center axis is a vertical axis, the first end of the tubular element (101) is the lower end of the tubular element (101), and the second end of the tubular element (101) is the upper end of the tubular element (101).

In preferred embodiments of the photobioreactor system of the present invention, the tubular element (101) is helically coiled around the center axis, forming a helix having an overall conical shape.

In preferred embodiments, the bioreactor system of the present invention further comprises a stabilizing framework (105, 106, 107), preferably a tension-based flexible stabilizing framework (105, 106, 107), supporting the cultivation vessel, wherein the stabilizing framework is configured to mount the photobioreactor system in a hanging manner to a support. Preferably, the stabilizing framework (105, 106, 107) comprises an array of stiffening rings at the upper edge of the helix (106) that is attachable to the support with flexible brackets (105) and serves as a holder for vertical side braces (107) to which the tubular elements (101) are attached. It is preferred that the array of stiffening rings comprises at least one ring (106a) as holder for the vertical side braces (107). More preferably, the array of stiffening rings comprises at least two stiffening rings, wherein a first stiffening ring is attachable to the support, a second stiffening ring is attached to the first stiffening ring, and the second stiffening ring carries the tubular elements (101). Yet more preferably, the array of stiffening rings further comprises additional rings as carriers for the light fixture (106b) and/or a global scaffold ring (106c) that serves for the attachment of 106a, 106b as well as the carrier for the equalization reservoir (201), the fluid return elements (202) and the aeration pump (203).

In preferred embodiments, the photobioreactor system of the present invention further comprised an aeration pump (203) configured to provide gas into said gas inlet (108).

In preferred embodiments, the photobioreactor system of the present invention operates without any circulation pumps, *i.e.,* said system does not comprise any circulation pumps.

In alternative embodiments, the photobioreactor system of the present invention further comprises one or more circulation pump(s) (204) configured to control the flow rate of the culture medium independently of the pressure and buoyancy of the gas from the aeration pump (203).

In preferred embodiments, the photobioreactor system of the present invention further comprises a secondary air inlet that is integrated into the equalizing reservoir (201), wherein said secondary air inlet has a lower gas flow rate compared to the gas flow rate of the at least one gas inlets (108).

In further preferred embodiments, the photobioreactor system of the present invention further comprises inlet (205) and outlet valves (206) for the addition or removal of culture medium. In yet further preferred embodiments, the photobioreactor system of the present invention further comprises one or more sensor units for measuring optical density, pH value, and/or chemical composition of the culture fluid. In yet further embodiments, the photobioreactor system of the present invention further comprises one or more gas outlet valves (207) to allow excess gas to escape the system.

In specific embodiments, in the photobioreactor system of the present invention, the cultivation vessel comprises one or more additional tubular elements (101) extending helically around a center axis, wherein the diameter (102; 103) of the helix thus formed continuously increases as the tubular element extends along the center axis. In preferred embodiments, the tubular elements (101) are arranged (i) as a helix, e.g. a double helix, in which the ascending helices are alternately stacked, or (ii) in multiple layers. In such embodiments, the tubular elements (101) can be connected in series or connected in parallel. Further, in such embodiments, the photobioreactor system of the present invention further comprises at least one or more additional gas inlets (108) for each of the tubular elements (101).

In further embodiments, the photobioreactor system of the present invention further comprises one or more additional fluid return elements (202) and/or one or more additional lighting fixtures (110).

Concerning the lighting fixtures used in the photobioreactor system of the present invention, in specific embodiments, these lighting fixtures can be disposed in between tubular element helices and/or in between tubular element layers.

In a specific embodiment of the photobioreactor system of the present invention, the equalizing reservoir (201) and the at least one fluid return elements (202) are combined and are located in the center of the bioreactor system, thereby forming a centrally arranged equalization reservoir that also serves as a fluid return element.

In preferred embodiments, in the photobioreactor system of the present invention, the fluid return elements (202) and/or the lighting fixtures (110) are entirely surrounded by the tubular elements (101). In related preferred embodiments, in the bioreactor system of the present invention, the aeration pump (203) and fine particle filter, the equalizing reservoir (201), the one or more circulation pumps (204), the inlet (205) and outlet valves (206), and/or the sensor units are located inside or above the tubular element (101). More preferably, all of said elements are located inside or above the tubular element (101).

In preferred embodiments, in the photobioreactor system of the present invention, the equalizing reservoir (201) is configured to provide exchange with the ambient air via a fine particle filter.

In preferred embodiments, the lower, smallest helix diameter (102) is from about 0.10 m to about 5.00 m, more preferred from about 0.25 m to 2.00 m, e.g. about 0.50 m. In further preferred embodiments, the upper, largest helix diameter (103) is from about 0.20 m to about 15.00 m, more preferred from about 2.00 m to about 6.00 m, e.g. about 6 m. In yet further preferred embodiments, the height (104) of the helix is from about 0.30 m to about 8.00 m, more preferred from about 1.50 m to about 4.00 m, e.g. about 3 m. In yet further preferred embodiments, the number of helix turns is from about 10 to about 150 helix turns, more preferred from about 20 to about 60 helix turns.

In other preferred embodiments, the outer diameter of a tubing forming the tubular element (101) is from about 0.01 m to about 0.5 m, more preferably from about 0.02 m to 0.1 m, most preferably from about 0.03 m to about 0.06 m. In further preferred embodiments, the wall thickness of a tubing forming the tubular element (101) is from about 0.01 mm to about 25 mm, more preferably from about 1 mm to about 15 mm, most preferably from about 2 mm to about 8 mm. In a specific exemplary embodiment, the tubing outer diameter is 0.05 m, and the wall thickness is 5 mm leading to an inner tube diameter of 0.04 m.

In preferred embodiments, the tubular element (101) and/or the fluid return element (202) are made of a material, selected from the group consisting of silicone rubber, silicone rubber composites, PVC (polyvinylchloride), polyethylene (PE), HDPE (high density polyethylene), PP (polypropylene), PET (polyethylene terephalate), acrylate (plexiglas), fiberglass (glass-reinforced plastic (GRP)) polyester, fiberglass (GRP), glass, and combinations thereof.

Further, it is preferred that at least the tubular element (101), the fluid return element (202), and/or the equalizing reservoir are made of a transparent or translucent material.

In preferred embodiments, the photosynthetic microorganisms cultivated in the photobioreactor system of the present invention are selected from the group consisting of eukaryotic micro-algae and cyanobacteria.

Further, as used herein the term "gas" preferably refers to carbon dioxide (CO₂) or a CO₂-containing gaseous mixture, preferably air. Further, said gaseous mixture can further contain carbon monoxide (CO), ammonia (NH₃) and other gaseous nitrogen and sulfur compounds.

In preferred embodiments, the bioreactor system of the present invention is operated in a continuous mode, *i.e.,* the nutrient culture solution circulates in a constant flow through the entire volume of the bioreactor, including the helical tubular element (101), the equalization tank (201), and the liquid return element (202). In continuous operation, the constant flow is induced by the rising gas and/or by additional circulating pumps. The flow velocity of the circulating medium flow depends strongly on the tube diameters and can be varied in wide ranges, depending on the application. As an example, a bioreactor comprised of a helical coiled tubing with 0.05 m inner diameter with a capacity of about 60 L is typically operated at a flow rate of 100 to 200 L/h for continuous cultivation of algae. To achieve such flow rates at this capacity by gas buoyancy alone, between 5 to 50 L of gas per minute are injected into gas inlet 108.

Generally, the photobioreactor systems of the present invention can be operated at flow rates of between 20 to 400 L/h, in combination with suitable tubing inner diameters and tubing capacities, e.g. at flow rates of between 50 to 300 L/h, 50 to 250 L/h, 100 to 200 L/h, or at about 150 L/h.

PMO that can be cultivated in the bioreactor systems of the present invention are not particularly limited and include any PMO the cultivation of which might be of interest. By way of example, PMO may be selected from the group consisting of eukaryotic micro-algae and cyanobacteria, wherein the cyanobacteria may be selected from the group consisting of cyanobacteria of the class *Cyanophyceae,* and/or the eukaryotic micro-algae may be selected from the group consisting of eukaryotic micro-algae of the classes *Chlorophyceae, Bacillariophyceae, Chrysophyceae, Prymnesiophyceae, Trebouxiophyceae, Eustigmatophyceae, Chlorodendrophyceae.* Further, PMO may be bacterial and algal strains derived from the above, as well as recombinant bacterial and algal strains derived from the above, and combinations thereof.

As specific examples, the cyanobacteria of the class *Cyanophyceae* may be selected from the group consisting of *Arthrospira* sp., *Spirulina* sp., and *Haematococus* sp. (e.g. *Haematococus pluvialis),* the eukaryotic micro-algae of the class *Chlorophyceae* may be selected from the group consisting of *Dunaliella* sp. (e.g. *Dunaliella tertiolecta* or *Dunaliella salina),* the eukaryotic micro-algae of the class *Bacillariophyceae* may be selected from the group consisting of *Chaetoceros* sp. and *Phaeodactylum* sp. (e.g. *Phaeodactylum tricornutum*)*,* the eukaryotic micro-algae of the class *Prymnesiophyceae* may be selected from the group consisting of *Isochrysis* sp.(e.g. *Isochrysis galbana),* the eukaryotic micro-algae of the class *Trebouxiophyceae* may be selected from the group consisting of *Chlorella* sp. (e.g. *Chlorella salina* or *Chlorella vulgaris), Nannochloris* sp., and *Coccomyxa sp.,* the eukaryotic micro-algae of the class *Eustigmatophyceae* may be selected from the group consisting of *Nannochloropsis* sp. (e.g. *Nannochloropsis salina),* and/or the eukaryotic micro-algae of the class *Chlorodendrophyceae* may be selected from the group consisting of *Tetraselmis* sp. (e.g. *Tetraselmis suecica* or *Tetraselmis chuii*)*.*

The PMO cultivated in the photobioreactor systems of the present invention may be grown singly or as a mixed species culture. In specific embodiments, the PMO are grown in a mixed community with one or more additional organisms, selected from the group of marine and freshwater microalgal species, such as from the group of diatoms (e.g., *Bacillariophytina, Coscinodiscophytina).* In another specific embodiment one or more additional organisms are selected from the group consisting of endosymbionts, rotifers, amoebae, ciliates, flagellates, suitable members of the kingdom eukarya, bacteria or archaea, or combinations thereof. Suitable endosymbionts in this respect may be selected from the group consisting of bacterial endocytobionts, endosymbiotic ciliates, endosymbiotic purple bacteria, and endosymbiotic green algae. Suitable members of the kingdom eukarya include, for example, free or submersed floating aquatic plants, saltwater or freshwater fish, or crustaceans.

In a second aspect, the present invention relates to an array comprising two or more individual photobioreactor systems of the present invention, wherein the individual photobioreactor systems are connected and integrated into a serial or parallel circuit.

In a third aspect, the present invention relates to a method of air filtration in an animal house, comprising the step of operating one or more photobioreactor systems of the present invention or an array of the present invention in said animal house.

In a related fourth aspect, the present invention relates to the use of the photobioreactor system of the present invention or of an array of the present invention for air filtration in an animal house.

In a fifth aspect, the present invention relates to a method for the cultivation of photosynthetic microorganisms, comprising the step of operating one or more photobioreactor systems of the present invention or an array of the present invention in an animal house.

In a related sixth aspect, the present invention relates to the use of the photobioreactor system of the present invention or of an array of the present invention for the cultivation of photosynthetic microorganisms.

In a seventh aspect, the present invention relates to a method for the conversion of waste gases produced in an animal house to biomass, comprising the step of operating one or more photobioreactor systems of the present invention or an array of the present invention in said animal house.

In a related eighth aspect, the present invention relates to the use of the photobioreactor system of the present invention or of an array of the present invention for converting waste gases produced in an animal house to biomass.

As used herein, the term "waste gases" includes carbon monoxide (CO), carbon dioxide (CO₂), water (H₂O), ammonia (NH₃) and a variety of volatile nitrogen and sulfur components, as well as any gas mixtures containing the same, e.g. air (such as ambient air) containing elevated amounts of CO, CO₂, NH₃, volatile nitrogen and sulfur components, and fine dust and other particulate matter.

In the above seventh and eighth aspect of the present invention, the conversion of waste gases produced in an animal house to biomass is effected by the operation of photobioreactor system of the present invention in said animal house, i.e., said waste gases are introduced into the bioreactor system and metabolized by organisms, e.g. photosynthetic microorganisms, growing in said bioreactor system, said organisms representing the generated biomass.

In all of the above aspects, the photobioreactor system is as described herein.

Closed bioreactors (vertical columns, flat plates, tubes, etc.) are known in the art for their excellent ability to control sterility as well as all parameters affecting growth, such as temperature, pH, light intensity and duration, O₂/CO₂. and nutrient concentration. In this way, these systems allow the continuous cultivation of a wide variety of species. However, their application within conventional, industrial animal houses has not been described.

The present invention provides a bioreactor system for the cultivation of PMO that can be operated in conventional animal houses in order to use the emissions generated there for the production of value-added compounds. As will be evident from the following, the unique design of the bioreactor system described herein allows it to combine a controlled environment (light exposure, impurities, stable pH, and CO₂ supply) for the cultivation of microorganisms with a physical and functional fit for integration into animal housing.

### Helical tube construction (tubular element)

To meet the above-described demands, the present inventors have developed a cultivation system that uses a helically wound tube construction as the cultivation vessel, with the lower diameter of the spiral being smaller than the upper diameter, resulting in an overall conical shape. The cultivation vessel is almost arbitrarily scalable in terms of its individual size and culture volume by changing the diameter and number of turns of the helix and/or the diameter of the tubing. Typical, non-limiting examples of the helix dimensions are listed in Table 1.

**Table 1: Dimensions of the helical tube construction**

| Tubing internal Diameter [m] | Tubing external Diameter [m] | Angle α^{a)} [°] | Lower Helix Diameter (measure d at tube center) (102)^{b)} [m] | Upper Helix Diameter (measured at tube center) (103)^{b)} [m] | Vertical Space between tubes [m] | Number of Helical Turns | Height (104)^{b)} [m] | Volume [L] |
|---|---|---|---|---|---|---|---|---|
| 0.03 | 0.04 | **25** | 0.5 | 2.0 | 0.0 | 44 | 1.61 | 122 |
| 0.03 | 0.04 | **65** | 0.5 | 2.0 | 0.0 | 20 | 0.35 | 55 |
| 0.03 | 0.04 | **45** | 0.5 | 2.0 | 0.0 | 26 | 0.75 | 72 |
| **0.04** | 0.05 | 45 | 0.5 | 2.0 | 0.0 | 21 | 0.75 | 103 |
| 0.04 | **0.07** | 45 | 0.5 | 2.0 | 0.0 | 15 | 0.75 | 74 |
| **0.05** | 0.06 | 45 | 0.5 | 2.0 | 0.0 | 17 | 0.75 | 131 |
| **0.06** | 0.07 | 45 | 0.5 | 2.0 | 0.0 | 15 | 0.75 | 166 |
| **0.07** | 0.08 | 45 | 0.5 | 2.0 | 0.0 | 13 | 0.75 | 196 |
| **0.08** | 0.09 | 45 | 0.5 | 2.0 | 0.0 | 11 | 0.75 | 217 |
| **0.09** | 0.10 | 45 | 0.5 | 2.0 | 0.0 | 10 | 0.75 | 250 |
| 0.04 | 0.05 | 45 | 0.5 | **5.0** | 0.0 | 63 | 2.25 | 683 |
| 0.04 | 0.05 | 45 | 0.5 | **10.0** | 0.0 | 134 | 4.75 | 2,775 |
| **0.50** | 0.55 | 45 | 0.5 | 10.0 | 0.0 | 12 | 4.75 | 38,820 |
| 0.50 | **0.51** | 45 | 0.5 | 10.0 | 0.0 | 13 | 4.75 | 42,050 |
| 0.50 | 0.51 | 45 | 0.5 | 10.0 | **0.51** | 16 | 4.75 | 19,410 |

| | | | | | | | | |
|---|---|---|---|---|---|---|---|---|
| ^{a)} Angle α between vertical side braces and helix axis is indicated in Figure 4 ^{b)} Numbers in brackets refer to graphical illustrations in Figure 1 | | | | | | | | |

Preferred dimensions for the lower helix diameter (102) range from 0.10 to 5.00 m, more preferred from 0.25 to 2.00 m. In one particular embodiment, the lower helix diameter is about 0.50 m.

Preferred dimensions for the upper helix diameter (103) range from 0.20 to 15.00 m, more preferred from 2.00 to 6.00 m. In one particular embodiment, the upper helix diameter is about 6 m.

Preferred dimensions of the helix height (104) range from 0.30 to 8.00 m, more preferred from 1.50 to 4.00 m. In one particular embodiment, the helix height is about 3 m.

The number of helix turns is determined by the lower and upper helix diameters, the helix height and the distance between the individual turns arranged one above the other. Since in a preferred embodiment the distance between the individual turns arranged one above the other is as small as possible (see below), preferred numbers of turns result between 10 and 150 or more preferably between 20 and 60.

The preferred dimensions of the tubing used for the helical construction are an outer diameter between 0.01 and 0.5 m, preferably between 0.02 and 0.1 m and most preferably between 0.03 and 0.06 m. The wall thickness of the tube is between 0.00001 and 0.025 m, preferably between 0.001 and 0.015 m and most preferably between 0.002 and 0.008 m. The inner diameter of the tube, which ultimately determines the culture volume, is the difference between the outer diameter minus twice the wall thickness. In one particular embodiment, the tube outer diameter is 0.05 m, and the wall thickness is 0.005 m leading to an inner tube diameter of 0.04 m.

In a particular embodiment, the helix of the tubular element (101) is arranged as a double helix in which two ascending helices are alternately stacked. Hereby, a fluid volume element first ascends in one of the two helices into the equalizing reservoir (201), is guided down into the second helix by a first fluid return element (202), ascends again into the equalizing reservoir (201) and is guided back into the first helix by a second fluid return element (202). This arrangement increases the pitch of the helix so that a better lift is achieved by the pumped-in gas.

In another embodiment, the configuration of the double helix is such that a fluid volume element first ascends into the equalization reservoir (201) in one of the two helices and from there is returned to the inlet of the first helix either through the second helix or the fluid return element (202). In a particularly preferred embodiment, two parallel rising fluid volume elements are created at two gas inlets (108) by the injected gas flow, forced into the two stacked helixes and recombined in the equalization reservoir (201). All these arrangements increase the pitch of the helix so that better lift is achieved by the gas being pumped in.

In a specific embodiment, an array comprising two or more individual photobioreactor systems are connected and integrated into a serial or parallel circuit. In a preferred embodiment, two or more photobioreactor systems are linked in fluid connection into a serial circuit by arranging multiple tube helices (101) of different diameters into each other so that they form multiple layers of tube helices (101) within the photobioreactor in the manner of a Russian Matryoshka doll. In this embodiment a lighting fixture (110) is disposed between two adjacent layers of tubular elements (101), allowing for a higher density of tubes per cubic meter and thus more efficient use of space.

### Tubing materials

The tube used for the helix construction can be made of a variety of transparent or translucent materials that allow light to pass through the walls. When transparency or translucency (semi-transparent) is mentioned in connection with the present invention, it means that at least 30%, even 60%, further even 85 to 90% and sometimes even ≥ 95% of the light reaching the side wall of the tube passes through it.

The skilled person is aware of a wide variety of materials that meet the above criteria. Non-limiting examples include glass-, plastics- or polymer-based materials as well as composites thereof. In addition to the transparency or translucency of the tubing material, the mechanical properties (flexibility, stiffness, deformability), the permeability to gases and fine particulate matter as well as biocompatibility/bioadhesiveness must also be considered. Typical, non-limiting examples of materials suitable for manufacturing the helix construction according to the invention are listed in Table 2.

**Table 2: Tubing materials for the helical photobioreactor construction**

| **Material** | **Tensile Strength (GPa)** |
|---|---|
| Silicone Rubber | 0.00015 - 0.17 |
| Silicone Rubber Composites | <0.00001 - >45 |
| PVC (polyvinylchloride) | 0.003 - 0.01 |
| Polyethylene (PE) | 0.7 |
| HDPE (High Density Polyethylene) | 0.8 |
| PP (Polypropylene) | 1.5-2 |
| PET (Polyethylene Terephalate) | 2 - 2.7 |
| Acrylate (Plexiglas) | 1.5-3 |
| Fiberglass (GRP) Polyester | 17 |
| Fiberglass (GRP) | 45 |
| Glass | 90 |

In one embodiment, a tubing made of transparent silicone rubber is used for the photobioreactor according to the invention. Here, the mechanical stability of the helix construction can be specifically influenced by using different silicone rubber composite materials. The tensile strength of unreinforced silicone rubber is typically lower than 0.4 MPa, and the tensile strength of highly transparent silicone modified materials is up to 1.5 MPa. By using silicone-based composites containing other organic polymers and/or ceramic particles, transparent materials with tensile strength of 6 MPa and more can be produced. Even silicon rubber materials with >40 GPa are known. Since the overall mechanical stability and physical robustness of the helix construction is also influenced by the tensile strength of the tubing material due to the tensile force-based suspension according to the invention, it can be tailored for special applications in animal housing.

In another embodiment, a tubing made of transparent, inelastic material, such as acrylate or glass, is used for the photobioreactor according to the invention. Although this inelastic material increases the rigidity of the individual helical turns and thus of the overall structure, this high rigidity also results in a lower physical load-bearing capacity of the overall structure, since mechanical shocks, for example from animals or maintenance personnel, cannot be flexibly absorbed. To compensate for this effect, sections of helix windings made of the inelastic material can be connected to each other by flexible flanges so that mechanical shocks can be absorbed and cushioned by the overall structure.

In another embodiment, the photobioreactor system according to the invention is made of sections of helical turns consisting of different materials and connected to each other by flexible flanges. For example, sections made of a flexible material, e.g. silicone rubber, are connected to sections made of an inelastic material (acrylates) by flexible flanges. Connecting sections with flanges can also be used to accomplish integration of sensor units (e.g., for measuring optical density, pH value, or chemical composition of the culture fluid) or inlet or outlet tubes (e.g., for adding nutrients to the culture medium or for taking analytical samples, respectively) into the helical photobioreactor. Thus, by using sections connected with flanges, a customized overall design can be produced that has an optimal fit (e.g., cushioning of mechanical shocks, integration of sensors, or supply and discharge of other volume flows) to meet the requirements in specific animal house applications.

In another embodiment, the tubing for photobioreactor systems according to the invention are made of different materials that differ in terms of their interaction potential with the gas and the cultivation medium. It is known to the person skilled in the art that the different tube materials show large differences with respect to wettability with water, gas permeability, or bioadhesiveness. For example, tubes made of silicone rubber are very hydrophobic, gas permeable, and less susceptible to micro-algae adhesion than tubes made of PVC, which are also hydrophobic but not gas permeable.

In a preferred embodiment, the tubing for photobioreactors according to the invention is made of silicone rubber materials.

In another embodiment, the spiral wound cultivation tube consists of sections of tubes made of different materials, such as but not limited to silicone, acrylate, glass etc.). The various tube sections are connected by use of flanged sleeves. These flanged sleeves not only allow to connect tube sections made of different materials, but also enable physical integration of sensors or sampling valves for convenient in-line analysis or sample extraction of the culture medium. As an example of the latter, the flanged sleeves allow outlet and inlet lines to be connected to any point on the helical tube reactor to take samples for analysis or to feed nutrients. Thus, this helical tubing configuration through flanged sleeves allows a fast and interference-free analysis of culture conditions and suitability of material properties, for example to test differences in bioadhesion or gas permeability between different tubing materials.

The nature of the tubing material influences the formation of biofilms at the interface between the tubing and the culture solution. Both the formation and prevention of biofilms can be beneficial for certain applications. For example, highly pure, sterile suspension cultures of microalgae are required for the production of pharmaceutical products, so biofilm formation is undesirable here. In contrast, in the production of biomass for animal feed in open pond systems, biofilms are known to positively influence the ecological stability of the production cultures. It is also known to those skilled in the art that complex microbial communities have a high potential for applications in biotechnology. In order to exploit the potential of complex mixed microbial consortia for biomass and metabolite production, in one embodiment the bioreactor according to the invention is equipped with tubing materials that favor colonization with bacterial biofilms. For this purpose, the skilled person is familiar with a variety of materials with suitable surface properties. These range from solid microscale smooth and microscale rough materials (silicone, PVC, PET) to microporous foams, fibrous and composites of fabricated and natural materials.

### Stabilizing framework

The helical photobioreactor systems according to the invention are preferably attached to the ceiling of the animal house with a tension-based, flexible stabilizing framework. This self-supporting structure is not only easy to integrate into existing barns but is also very resistant to absorb physical shocks from the animals or farm personnel, thus preventing mechanical damage to the reactor and its internal operating elements (lighting fixtures, pumps, valves, return tube).

The key elements of the tension-based flexible stabilizing framework comprise the array of stiffening rings (106) that is attached to the ceiling with several flexible brackets (105) and serves as holder for the vertical tubing braces (107) and the lighting fixture (110). Various non-limiting embodiments of the bioreactor according to the invention are detailed below.

### Stiffening rings (106) and flexible brackets (105)

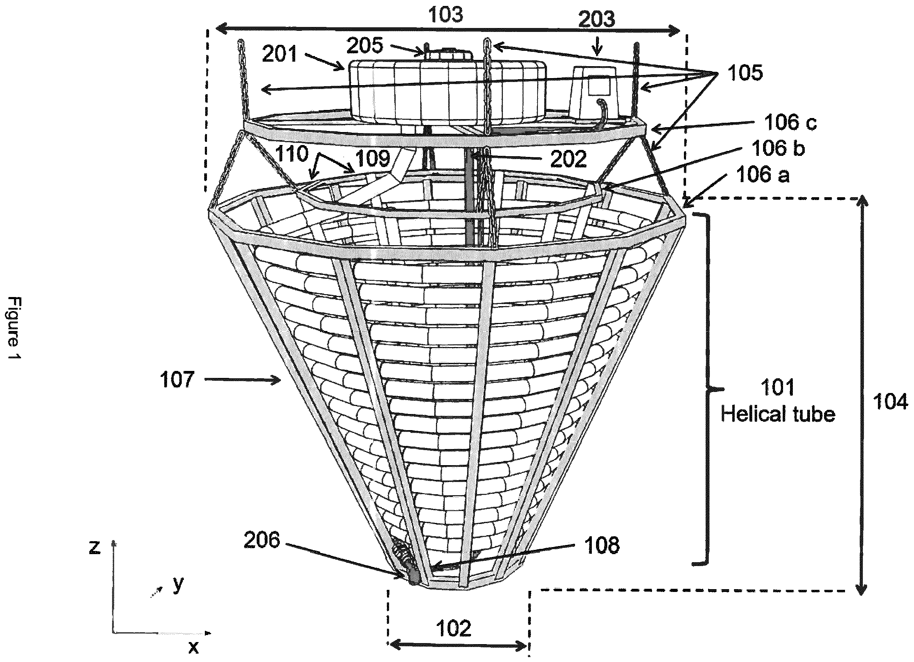

The reactor and the light fixture inside can be suspended in various ways. In a preferred embodiment, the suspension is made by one or more stiffening and shaping elements. These shaping elements can consist of the so-called stiffening rings (106, in Figure 1) but they can also consist of existing structures at the erection site by suspending the reactor directly from a sufficiently load-bearing stable ceiling via any number of circularly arranged fastening hooks. Thus, it is possible to suspend the reactor directly from existing structures at the erection site without a stiffening ring using circularly arranged suspensions. Advantageously, the suspension is made by using a central stiffening ring between the roof at the erection site and the reactor. Equally advantageous is the use of several stiffening rings between the roof at the erection site and the reactor, whereby each stiffening ring can be assigned a special equipment carrier function (see 106a, 106b, 106c in Figure 1).

The stiffening rings can be designed in various different ways. If one or more stiffening rings are to be used, they can be made of a variety of different materials, such as alloys of steel, aluminum, titanium, and other metals. Advantageously, lightweight materials known to the skilled person, such as reinforced and non-reinforced plastics, or natural materials, such as wood, bamboo, or composites accessible therefrom, are used.

The shape of the stiffening ring is decisive for the external shape of the reactor. Any polygonal shape can be realized by suitably shaped stiffening rings.

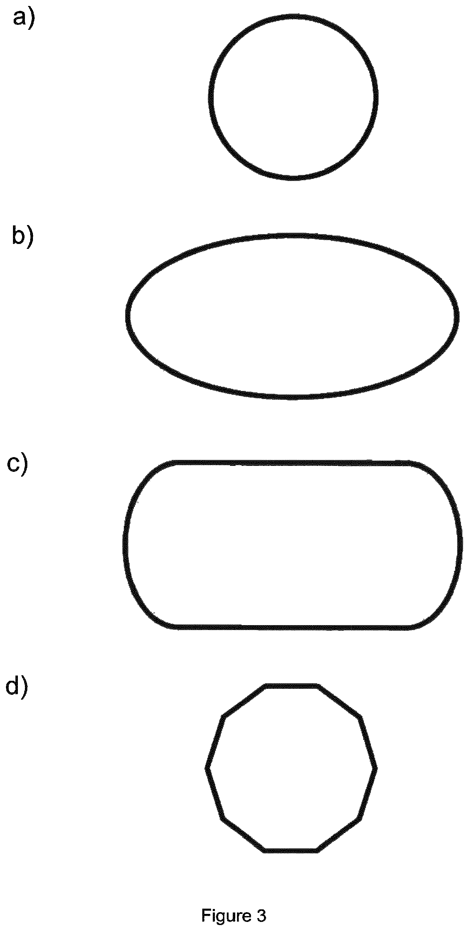

Preferably, the shape of the stiffening ring is circular, oval or the resulting shape of a cut circle, which is extended by parallel sides of any length. Possible shapes of the stiffening rings are illustrated in Figure 3.

If one or more stiffening rings are used, they can have different profile shapes depending on their size and the resulting load input. The profile shape is a cross-section of the stiffening ring profile. In one embodiment, the profile shape of the stiffening rings is realized as a circle, rectangle, square, oval, triangle, or polygon. In another embodiment, more elaborate profiles are realized that contain additional strut and/or truss profiles to compensate for increased load inputs from the reactor.

The suspension or connection of the stiffening ring to existing roof and/or ceiling structures at the erection site can be carried out in different ways. If one or more stiffening rings are used as a stiffening and shaping element, in a preferred embodiment the stiffening rings are connected to the load-bearing roof and/or ceiling structure of the erection site in order to be able to suspend the reactor safely and stably from the ceiling structure.

In another embodiment, this connection is made by screwing or bolting the stiffening ring(s) directly to a load-bearing roof, ceiling, or support structure. In another embodiment, the stiffening ring or rings are welded directly to a load-bearing roof, ceiling, or support structure.

In a preferred embodiment, the one or more stiffening rings are connected to a load-bearing roof, ceiling, or support structure by a number of n flexible brackets (105, in Figure 1). The flexible brackets can be of different lengths, made of different materials and of different shapes. The flexible brackets are spacers and can be of different lengths, materials, and shapes. In a preferred embodiment, the flexible brackets are in the form of ropes, chains, or rigid fasteners, such as a linkage. Also preferably, the flexible brackets are made of lightweight materials made of metals, plastics, synthetic fibers, natural fibers, or composites of these materials.

In another embodiment, the connection between the flexible brackets as spacing connecting elements, the roof or ceiling structure and the stiffening ring(s) is made by using bolted or welded hooks, eyes, or rings into which the flexible brackets can be hooked. It is also preferred that the flexible brackets be bolted or welded directly to the roof or ceiling structure and the stiffening ring(s) as spacer connecting elements.

In a particularly preferred embodiment, the suspension of the reactor consists of a stiffening ring of the reactor (106a, in Figure 1) and a further stiffening ring as a support for the luminous body (106b), which are jointly suspended from a further stiffening ring (106c). In this case, the stiffening ring 106c also serves as a support platform for the equalizing reservoir (201) and an aeration pump (ventilation compressor, 203), and also functions as the top element of the structure, which can be attached at any number of points via flexible brackets (105) to the ceiling or a support frame at the installation site.

In a likewise preferred embodiment, the helical tube construction of the reactor is terminated by a lower stiffening ring. The same specifications and options apply to this lower stiffening ring as to the upper stiffening rings in terms of shape, profile form and use of material.

In another preferred embodiment, a number of n further central stiffening rings are integrated between the upper and lower stiffening rings. These middle stiffening rings have a shape-directing and mechanically stabilizing function for the reactor and follow the same specifications and possibilities in terms of shape, profile form and material use as described for the upper stiffening rings.

### Vertical side braces (107)

The vertical side braces (107 in Figure 1) connect the upper and lower stiffening rings of the spiral tube and they serve as support elements for the reactor tubing. They can be made of various materials, although lightweight materials are preferred. Thus, in one embodiment, metals such as steel, aluminum or titanium are used. In a preferred embodiment, the vertical side braces are made of lightweight materials of reinforced and non-reinforced plastics, synthetic fibers, or natural fibers such as wood, bamboo or composites consisting thereof.

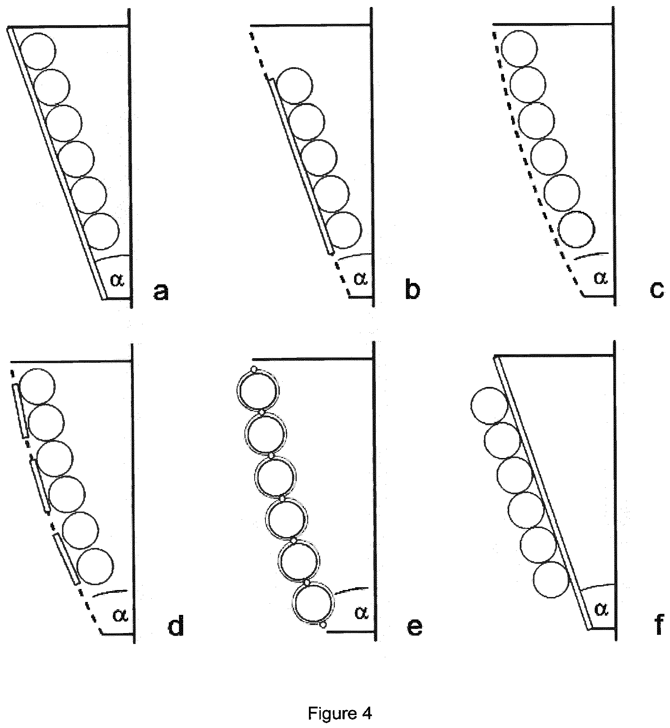

The vertical side braces can be connected to the stiffening rings in various ways in order to be used advantageously for the suspension of the reactor structure at the erection site. In one embodiment, the vertical side braces consist of a rigid, spacer profile that is directly and inflexibly connected to the stiffening rings (Figure 4a).

In a preferred embodiment, the vertical side braces consist of a rigid spacer profile connected to the upper and lower stiffening rings via a flexible, non-rigid element (Figure 4b). In this embodiment, the suspended reactor is highly resistant to external forces, such as impacts, because it can absorb them flexibly. The flexible elements can be chains, ropes, or elastic elements made of rubber or other elastomers. In another embodiment, the vertical side braces are connected to only one of the two stiffening rings via a flexible element and to the other via an inflexible, rigid element.

In another preferred embodiment, the vertical side braces consist entirely of flexible elements (Figure 4c). These flexible elements can be ropes, chains or connecting elements made of plastics, rubber, or other elastomers. In this configuration, the suspended reactor construction has a very high resistance to external impact forces, as it can flexibly absorb the mechanical forces acting on it.

In another embodiment, the vertical side braces consist of a number n of alternating rigid and flexible non-rigid spacer elements (Figure 4d). In this design, the suspended reactor is very robust against external forces caused by shocks, which it can absorb flexibly. The flexible and rigid elements used in this embodiment are typically made of the materials described in the embodiments above.

In another embodiment, the vertical side braces are short spacer rigid or flexible elements that have a clamp as a retainer at their upper and lower ends (Figure 4e). These connecting elements are attached at the top to the upper stiffening ring so that the first helix winding is attached to their lower side. These connecting elements are then fastened again to this first helix winding with their upper connection in order to hold the helix winding below with their lower side.

In another preferred embodiment, the vertical side braces consist of a rigid, spacer profile that is directly and inflexibly connected to the stiffening rings with the tubing placed outward with respect to the central helix axis (Figure 4f). Other preferred embodiments route the tubing outward with respect to the central helix axis by using flexible spacer profiles, such as those shown in Figure 4b to e.

Depending on the reactor design, the vertical side braces can be connected to the stiffening rings in various ways. In one embodiment, the vertical side braces are directly and firmly connected to the stiffening rings by welding, screwing, gluing, or clamping. In another embodiment, the vertical side braces are flexibly connected to the stiffening rings by being mounted via eyelets or hooks.

The vertical side braces serve as support elements for the tubing coils of the photobioreactor. Depending on the selected size of the reactor and the properties of the tubing, at least 2 vertical side braces are used in the reactor construct. Preferably 3 to 64, more preferably 4 to 32 and especially preferably 5 to 16 vertical side braces are used in the reactor construct.

In one embodiment, the vertical side braces are located internally with respect to the central axis of the helical tubular cone. In this embodiment, there is easy access to the helically wound tubing, allowing easy assembly and sampling. Also, a shock-absorbing effect results due to the elastic tube, which in certain applications in animal houses acts as a buffer against mechanical damage to the reactor structure by the kept livestock or care personnel.

In another preferred embodiment, the vertical side braces are located externally with respect to the central axis of the helical tubular cone. When the vertical side braces are positioned outside the tube cone, they provide additional protection of the reactor cone against mechanical shocks, and at the same time shading of the reactor tube by the luminous body, which is also located inside, is avoided.

The reactor tube can be attached to the vertical side braces in various ways. In one embodiment, there are loops or clips on the vertical side braces with which the tubing is held and guided along the vertical side brace. In another embodiment, the vertical side braces have hooks, open or closed clips, open or closed pipe clamps with which the tubing is attached to the vertical side brace and guided along. Particularly preferred is the use of clips or pipe clamps that can be reversibly opened and closed, thereby enabling easy assembly/disassembly of the reactor tubing in the suspended scaffold.

### Internal operational elements

The helical photobioreactor system according to the invention is configured in such a way that all necessary operational elements (pumps, valves, equalizing reservoir, lighting fixtures, return tube) are located inside or above the helical structure. This allows a zero-footprint design so that the entire floor area of the animal house is unrestricted for animal housing and cleaning. In addition, the flexible, self-supporting structure of the helix reactor protects the operating elements located inside from mechanical damage caused by physical contact with the animals or operating personnel.

### Gas Inlet, circulation pumps, and gas outlet

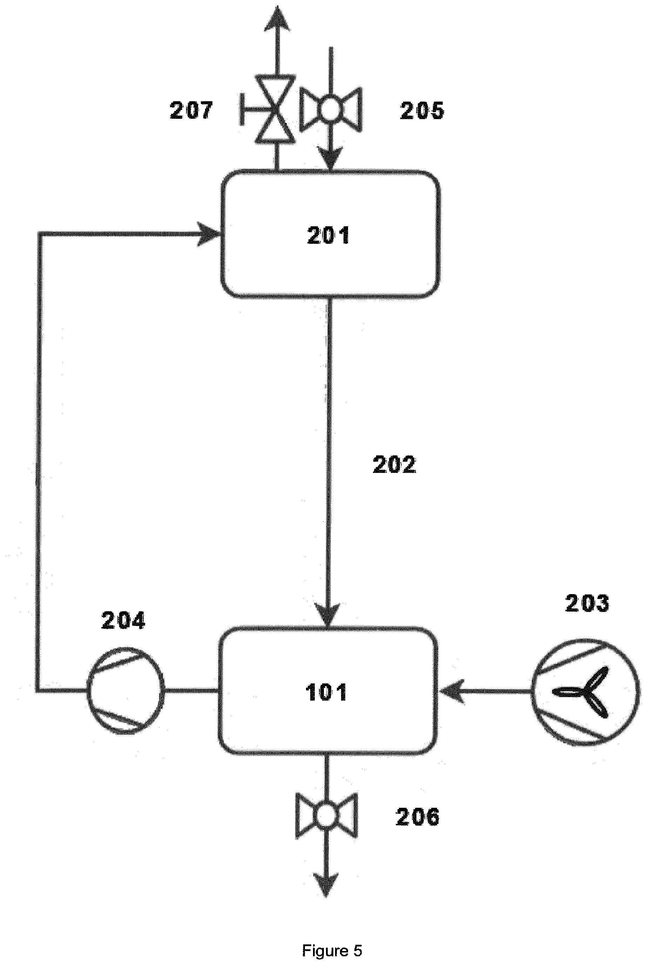

In one embodiment of the helical tube photobioreactor system according to the invention, an aeration pump (aeration compressor) is integrated above the helix (203, in Figure 1). From this aeration compressor, a gas hose leads into the gas inlet (108), which is located at the lower end of the helix and through which gas is continuously injected into the helical tube photobioreactor from below. The gas blown in and rising in the reactor causes the nutrient solution in the helical tube photobioreactor to be continuously circulated so that the gases required for growth, such as carbon dioxide, ammonia, methane, etc., are effectively introduced. The corresponding flow diagram is shown in Figure 5. Also shown in this figure is a gas outlet valve (207), which is used to discharge excess gas from the reactor system.

Depending on the space conditions of the installation site, the aeration compressor can be positioned at different locations. In one embodiment, the aeration compressor is positioned inside or below the conical tube helix. In such a positioning of the aeration compressor below the water column of the helical tube photobioreactor, a check valve is placed in front of the aeration compressor to prevent the aeration compressor from being flooded by the nutrient solution in case of a technical failure. In a preferred embodiment, the aeration compressor is positioned above the conical tube helix. Equally preferably, the aeration compressor draws in gas (e.g. ambient air) via a gas filter. For this purpose, a variety of filters are known to the skilled person, with which impurities such as particulate substances, microorganisms, or aerosols can be effectively removed in order to avoid undesired contamination of the nutrient solution in the helical tube photobioreactor.

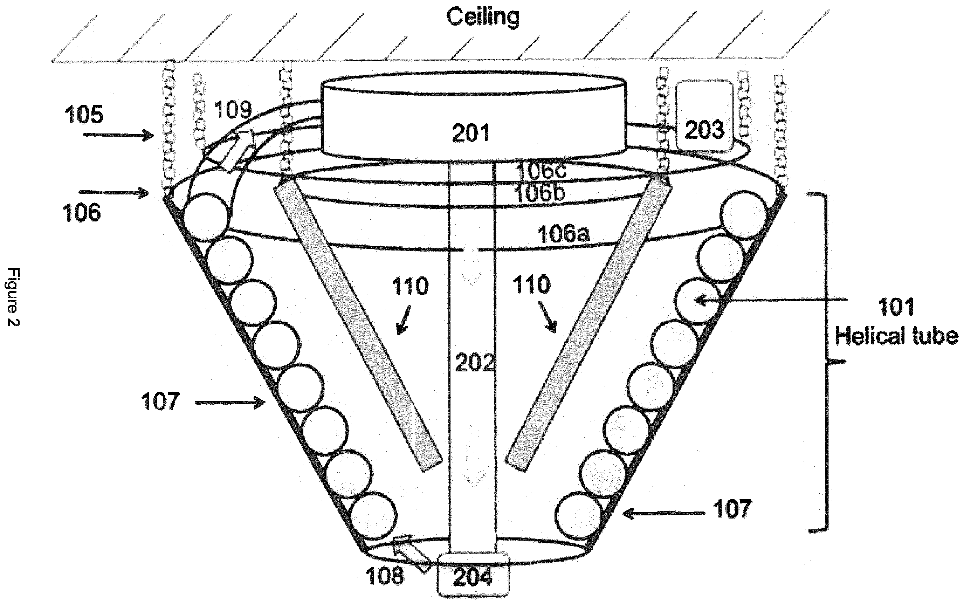

In specific embodiments, the photobioreactor system of the present invention operates without any circulation pumps, *i.e*., said system does not comprise any circulation pumps. In alternative embodiments, one or more circulation pumps (204, in Figure 2 and Figure 5) are used in addition to the circulation of the nutrient medium by the aeration compressor. Numerous variants of circulation pumps are known to the skilled person, such as rotary pumps or peristaltic pumps. These circulation pumps are integrated below, inside or above the conical tubing helix and allow the flow rates of the nutrient medium to be controlled independently of the pressure and buoyancy of the gas from the aeration compressor. Also, the circulation pumps allow to move the liquid volumes of only partially filled tubing reactors, for example to facilitate filling or cleaning.

In another embodiment, an additional secondary air inlet is integrated into the Equalizing Reservoir (201). This secondary air inlet has a lower air flow rate compared to the gas inlet (108) and ensures that the required carbon dioxide content within the tube system is kept high throughout the reactor without significantly affecting the flow characteristics within the reactor. This secondary air inlet is attached to the equalizing reservoir (201)/vertical return tube (202). It is particularly preferable to install the secondary air inlet at the lowest point of the equalizing reservoir (201 )/vertical return tube (202), as this creates buoyancy and air bubble-induced turbulence due to the inflowing air, which improves mixing of culture medium and prevents sedimentation of solids (e.g. cells, aggregated biomass) that could settle and accumulate in the equalizing reservoir (201 )/vertical return tube (202).

### Valves

In an embodiment of the helical tube photobioreactor system according to the invention, valves are integrated to facilitate the addition or removal of liquid quantities, harvesting processes, and/or the filling or emptying of the helical tube reactor. Preferably at least one, more preferably two valves are integrated into the reactor system. Particularly preferred are an inlet valve (205) and an outlet valve (206) that connect the photobioreactor to central supply lines (Figure 5). Also particularly preferred is the placement of the outlet valve (206) at the bottom of the spiral tube and the placement of the inlet valve (205) above the spiral tube. A large variety of valves can be used for this purpose, which are known to the skilled person. Non-limiting examples include two-way, three-way, and four-way valves that can be controlled manually or electronically. The valves are physically connected to the reactor circuit by methods known to the skilled person, such as flange, click, clamp, or even fixed adhesive connections.

In another embodiment of the helical tube photobioreactor system according to the invention, gas is permanently supplied to the reactor by a compressor, which is also injected into the reactor tube through a separate branching valve at the lower end of the helix.

In another embodiment, although the gas flow introduced by the compressor is already sufficient to realize an adequate reactor flow, a circulation pump is also integrated, which is connected to the helical tube bioreactor via a bypass. In this bypass solution, the pump is also connected to the helical tube bioreactor via a separate inlet and outlet valve each.

In a likewise preferred embodiment, the valves used are combined with a shutoff valve and/or a check valve to prevent backflow of the liquid in the reactor in the event of malfunctions or maintenance work.

### Equalizing reservoir

In an embodiment of the helical tube photobioreactor system according to the invention, the liquid flow from the reactor tubing is directed into a reservoir, which serves as an equalizing reservoir vessel (201) for the purpose of air and pressure equalization and also allows exchange with the outside to facilitate equilibration of oxygen- and CO₂-rich air. Due to the permanent inflow of gas from the lower inlet valve (108) into the helically wound reactor tube, the equalizing vessel is preferably located at the highest point of the reactor structure and connected to the lower part of the helically wound reactor tubing via the vertical return tube (202). Due to this arrangement, the culture liquid displaced by the gas from the helically wound reactor tube collects in the equalizing vessel and forms a growing liquid column there. As the liquid column in the equalizing vessel rises, a growing amount of medium is returned through the return tube to the beginning of the helical tubing. Depending on the strength of the compressor (aeration pump, 203), a liquid column of varying height forms until an equilibrium is reached between compressor output and returning medium. The water column then levels off at a constant height. In accordance with this behavior, the volume of the equalizing reservoir is preferably selected to be between 5 and 50%, preferably 15 and 40% and most preferably between 25 and 35% of the tube volume. Representative tube volumes are given in Table 1.

Since, in addition to the liquid, the gas blown in by the compressor also accumulates in the equalizing vessel, in a particularly preferred embodiment this vessel is not closed off but is connected to the surroundings via an air equalizer. In this embodiment, the equalizing vessel is provided with a gas outlet valve (207), which in turn is equipped with a gas filter to allow gas but no particulate matter into the interior of the reactor chamber. Numerous filter materials are known to the skilled person, which have defined filter performances and size exclusion properties in order to avoid contamination with e.g., fine dust or microorganisms.

Also known to the skilled person is a large number of filters with which airborne contaminants (e.g., toxic gases) can be removed from the ambient air. Particularly preferred in this embodiment are replaceable filters that are replaced at regular intervals. Also preferred in this embodiment is a sufficiently large opening in the equalizing vessel to allow it to be cleaned. This cleaning opening can be sealed airtight and watertight after closing, so that the gas exchange takes place exclusively via the filter. The equalizing vessel can be made of various materials known to the skilled person, including PVC, silicones, and other plastics, as well as glass, ceramics, metals, alloys, or composites thereof.

In another preferred embodiment, the equalization reservoir vessel (201) is combined with the vertical return tube (202) and is located in the center of the bioreactor. This creates a centrally arranged equalization tank that also serves as a return tube. It is also preferred that the equalization reservoir (201) and the vertical return tube (202), or alternatively the combined equalization vessel/return tube vessel, are made of a translucent or transparent material to ensure continuous and efficient illumination of the PMO throughout the reactor.

### Lighting fixtures

To enable the cultivation of PMO, the helical tube photobioreactor system according to the invention is equipped with a light source. In one embodiment, this lighting fixture/luminous body is located outside the conical tube helix, however, in a preferred embodiment, the lighting fixture/luminous body is positioned inside the conical tube helix.

In a preferred embodiment, the luminous body uses LEDs (light-emitting diodes) to generate light. LEDs can emit light with almost any spectral characteristics in terms of wavelength and intensity, and numerous embodiments are known to the person skilled in the art to provide LED illuminants with static or dynamic illumination characteristics in terms of wavelength, intensity, and/or irradiation time. In a particularly preferred embodiment, the photobioreactor according to the invention is equipped with an LED illuminant that allows variable wavelengths, irradiation intensities and irradiation times to be implemented in order to enable optimal growth of the PMO in the reactor.

In another embodiment, the photobioreactor according to the invention is equipped with an illuminant that employs conventional light sources, such as neon tubes, sodium vapor lamps, or mirror arrays for introducing natural sunlight.

In a preferred embodiment of the luminous body, a number of n tube LEDs are installed internally in the reactor helix so that each LED tube is arranged perpendicular to the helically ascending tube. In this way, the light energy is evenly distributed into the reactor chamber. In another preferred embodiment, using LED foils known to those skilled in the art, a uniformly illuminating cone is used, which is mounted internally parallel to the conical tube helix.

In another embodiment, a luminous body corresponding to the outer dimensions of the photobioreactor is positioned above the reactor on the inner surface of the upper stiffening ring. In further embodiments, a single central luminaire or flat luminaires are positioned at various locations inside the photobioreactor, or luminescent sheets are placed evenly on the inside of the reactor to cover its entire inside surface and deliver the same amount of light energy to each tubular region at each location in the reactor.

In another embodiment, thin, waterproof LED tubes are integrated into the interior center of the cultivation tube to allow effective use of the energy emitted by the LEDs without losses due to scattering and/or shadowing.

In another embodiment, further light sources are placed within the interior center of the bioreactor to illuminate the equalization reservoir (201) and the vertical return tube (202), or alternatively the combined equalization vessel/return tube vessel that are made of a translucent or transparent material, to ensure an efficient illumination of the PMO within the reactor.

### Return pipe (vertical return tube)

In one preferred embodiment of the helical tube photobioreactor system according to the invention, a central vertical return tube (202) is used to guide the liquid back from the equalizing reservoir to the lower end of the conical tube helix. This return tube thus enables the circulation of the medium and the conditions in which the PMO can be cultivated. Preferably, this recirculation line has the same diameter as the helix tube.

Likewise preferable, the vertical return tube (202) is combined with the equalization reservoir (201) to form a centrally arranged equalization tank that serves as the return tube. Particularly preferable is that the return tube (202), or alternatively the combined equalization vessel/return tube vessel are made of a translucent or transparent material, to ensure the continuous illumination of the PMO within the reactor.

In one embodiment of the bioreactor system according to the invention in which an additional circulation pump (204) for the culture medium is integrated, this circulation pump is positioned at the lower end of the vertical recirculation line.

In another embodiment, the central vertical return tube (202) is not a simple fall line but is designed as a separate secondary helically wound line. This return line may have a counter-rotational or co-rotational sense with respect to the rotation of the primary ascending helical coil charged with gas. In this case, it is preferred that the return line has the same direction of rotation as the primary ascending helix. This arrangement increases the pitch of the helix so that a better lift is achieved by the pumped-in gas.

### Applications

As shown in detail above, the helical photobioreactor system according to the invention can be tailored to provide a system for the cultivation of PMO that can be operated in conventional animal houses to use the emissions generated there for the production of value-added materials. Exemplary applications are explained using the non-limiting embodiments described below.

### Integration of helical tube photobioreactor in animal houses

In one embodiment, the photobioreactor according to the invention is mounted in an animal house for breeding fattening pigs. Depending on the height of the barn ceiling, preferred dimensions of the upper helix diameter are 6 to 12 m and of the lower helix diameter 1 to 2 m. The total heights and the number of turns result from the height of the barn ceiling. The photobioreactor is installed so that the lower edge of the helix is about 0.5 to 1.5 m above the floor of the cattle barn.

In another embodiment, the photobioreactor according to the invention is set up in an animal house for breeding laying hens. Depending on the height of the barn ceiling, preferred dimensions of the upper helix diameter are 6 to 12 m and of the lower helix diameter 1 to 2 m. The total heights and the number of turns result from the height of the barn ceiling. The photobioreactor is installed so that the lower edge of the helix is about 0.5 to 1.5 m above the floor of the poultry house.

### Helical tube photobioreactor in animal houses for cultivation of algae

In one embodiment, the photobioreactor installed in animal houses is used for continuous cultivation of micro-algae or cyanobacteria, e.g. *Spirulina* sp., *Chlorella* sp., or *Haematococus pluvialis,* by optimizing the light exposure times for the growth of the algae. When the optimal cell densities for the particular type of micro-algae or cyanobacteria are reached, approximately 50 to 80% of the culture medium is removed through the internal outlet 206, while the same volume of fresh medium is supplied through the inlet 205. To optimize such a harvesting process for isolating and processing algal biomass from the helical tube bioreactor, the process is ideally performed without interrupting reactor operation. This is achieved via the inlet (205) and outlet (206) valves that connect the photobioreactor to central supply lines (Figure 5), which allow the addition or removal of fluids without interrupting continuous operation. Through the central supply line, the biomass-loaded culture solution is transferred to a harvesting room separate from the animal house area by suctioning it using a pump located in the harvesting room. In the harvest room, the medium is then passed through a centrifuge to separate the algal biomass from the medium. The remaining medium with a significantly reduced algal biomass is enriched with fresh culture medium and returned to the helical tube bioreactor via a central supply line and the inlet valve (205). With the help of the medium exchange performed in this way, the cell density is reduced to 50-20% of the original density, allowing the PMO to re-enter the exponential growth phase. If necessary, the isolated cells are dried for further use, extracted, or simply used directly as wet feed for animal breeding.

In another embodiment, the photobioreactor system according to the invention installed in an animal house is used for culturing recombinant microorganisms that are genetically modified to produce valuable substances for the pharmaceutical or food industry. For example, micro-algae or cyanobacteria, such as *Spirulina* sp., *Chlorella* sp., or *Haematococus pluvialis,* are used for this purpose, and their growth conditions are optimized by tailored culture media and light exposure times. Here, the entire photobioreactor system is operated as a closed, sterile system, into which only emissions from the animal house, purified by sterile filters, are introduced. When the optimal cell densities for the respective type of microorganisms have been reached, about 50 to 80 % of the culture medium is exchanged using the internal inlet and outlet valves and the central supply lines, as described above. This ensures that the separation of the biomass and refreshment of the medium in the harvesting chamber is carried out under sterile conditions so as not to have to interrupt the continuous process. This procedure allows the cultivation process to be maintained for weeks and months. The separated biomass is processed using methods known to the skilled person in order to isolate the valuable components.

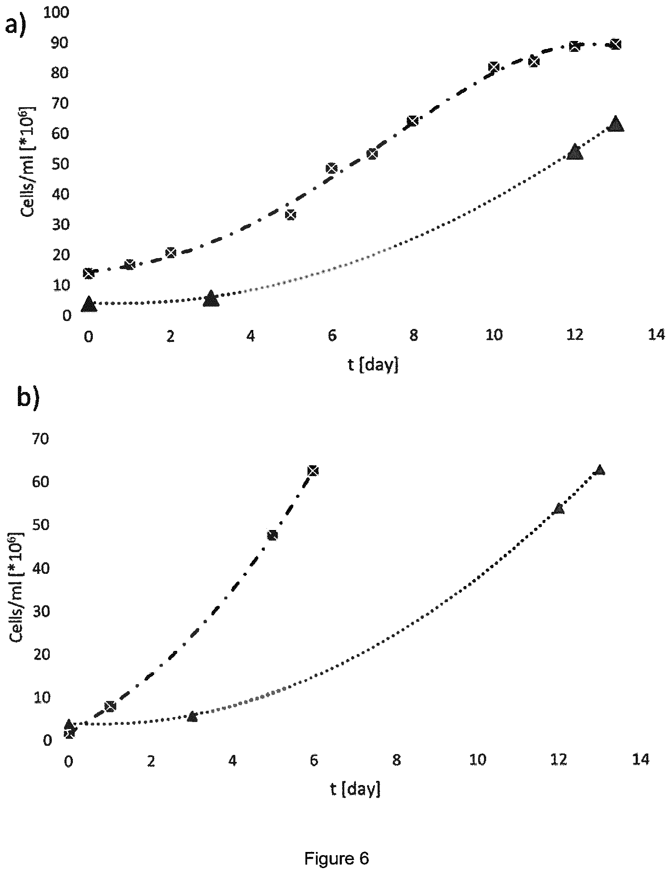

In one embodiment, *Chlorella vulgaris* micro-algae are cultured in a medium (1.2 g/l KNO₃ + 0.5 ml/I, Wuxal NPK (8/8/6) fertilizer) using either the helical tube photobioreactor system of the invention or conventional bag micro-algae cultivation methods using aerated transparent cultivation bags (Algabags from Algatec). The growth of the algae is monitored by sampling at different time points and microscopy-based quantification of the cell numbers. It is shown in Figure 6 that in the helical tube photobioreactor not only a significantly faster growth of the micro-algae takes place than in the bag system, especially in early phases of cultivation, but also significantly higher cell numbers are achieved.

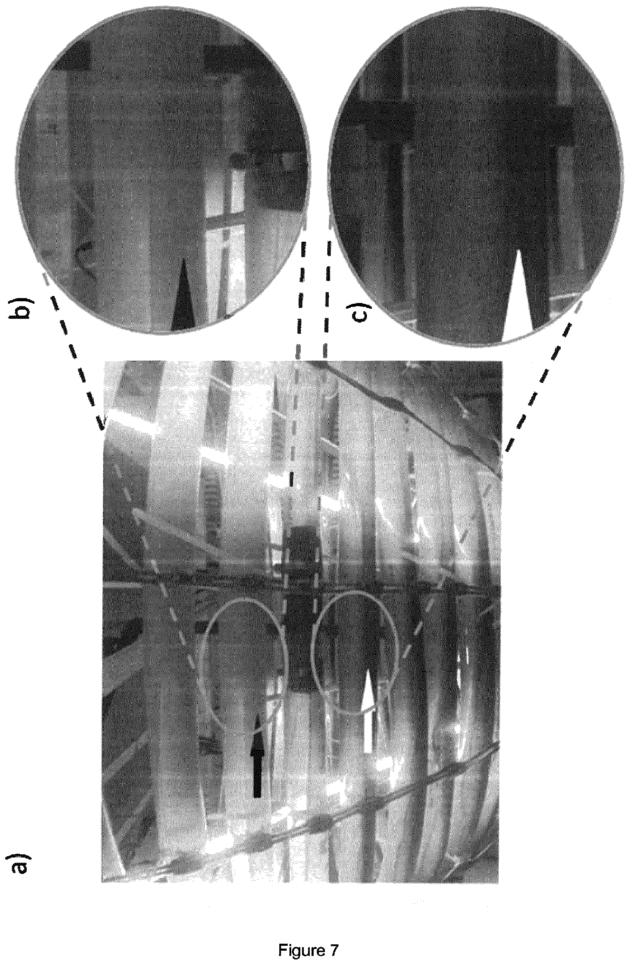

In another embodiment, the cultivation of micro-algae such as *Chlorella* sp. or *Spirulina* sp. is carried out in a helical tube photobioreactor system according to the invention. In this embodiment, the spirally wound cultivation tube consists of a series of tube segments connected with flanges. For example, two tubes made of PVC (bottom, white arrow) and silicone (top, black arrow) can be flanged together (cf. Fig. 7) to test differences in bioadhesion between different tube materials. The use of flanged sleeves allows not only to connect tubes made of different materials, but also to integrate sensors or sampling valves into these flanged sleeves for convenient in-line analysis of the culture medium. In the example shown in Figure 7, a strong deposition of a biofilm can be observed in the lower PVC part, while no significant biofilm adhesion occurred in the upper silicone part.

### Helical tube photobioreactor in animal houses for cultivation of mixed species

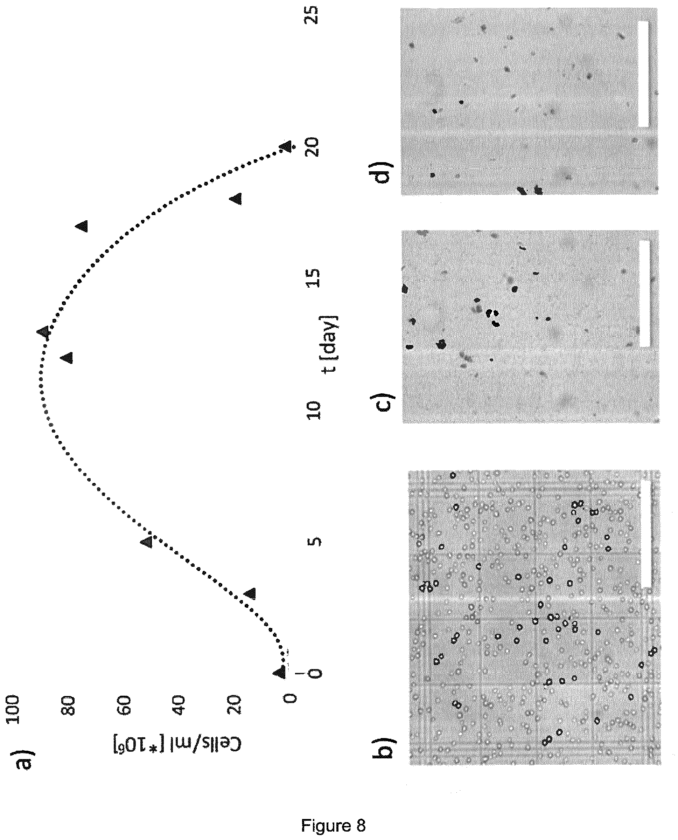

In another embodiment, the photobioreactor system according to the invention, which is installed in an animal house, is used for the cultivation of multispecies cultures consisting of at least two different species of microorganisms. Here, the two species can be freely selected from the kingdom of eukarya, bacteria or archaea, with at least one species belonging to the class of PMO. It is known to the skilled person that multispecies mixed cultures can be used to produce, for example, variations in the composition of biomass and metabolites that are not accessible with pure cultures. Therefore, cultivation of multispecies mixed cultures enables the production of value-added products that are not accessible with pure cultures. Dynamic interactions such as the formation of nutrient and metabolite gradients and ecological principles such as the predator-prey relationship between a predator (e.g., amoebae, ciliates, flagellates, rotifers) and a prey population (e.g., microalgae, bacteria, or other microorganisms) are at work in the formation of such multispecies cultures. By mimicking and exploiting these ecological interactions, stable multispecies cultures can be obtained that can be used to optimize the chemical composition of biomass produced in a reactor to access value-added products that cannot be produced with pure cultures. Figure 8 shows an example of model production of a multispecies culture of microalgae and rotifers, exploiting a predator-prey relationship in the coiled-tube photobioreactor of the invention to vary the biomass. When the optimal cell densities of the multispecies culture are reached, about 50-95% of the culture medium is removed and the reactor is refilled with fresh medium, as in the case of pure cultures, so that high cell densities are formed again based on ecological interactions. The cells obtained from harvesting the removed culture medium are separated and further processed using standard methods or used directly as feed for farm animals, fish, or insects.

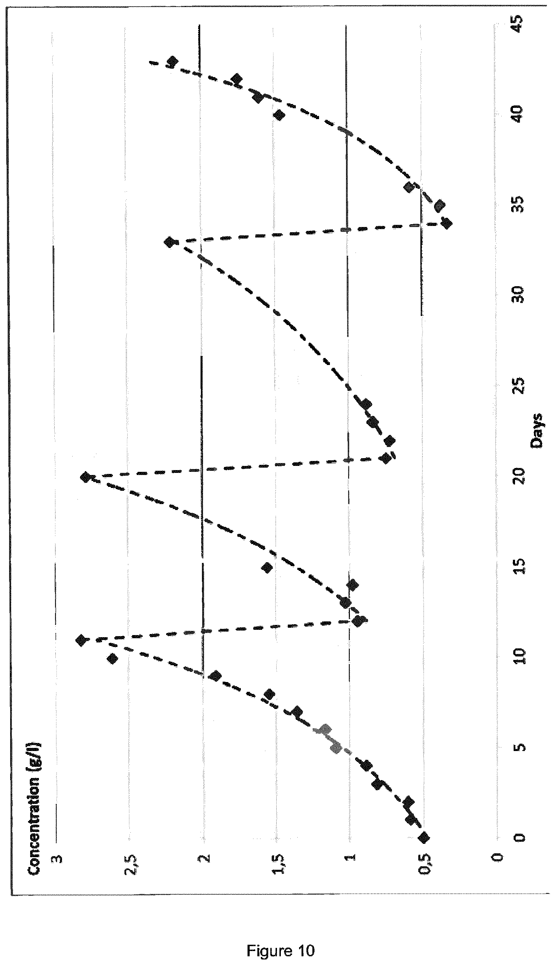

Figure 10 shows an example in which a mixed culture of *Spirulina* sp. microalgae *(Athrospira platensis)* and diatoms in a culture medium is continuously cultured in the helical tube photobioreactor system according to the invention. The growth of the *Spirulina* is monitored by determination of the concentration of the microalgae in grams (dry weight) per liter culture medium. Figure 10 shows that there is continuous rapid growth of microalgae in the helical tube photobioreactor, which can be reset to a low growth value by harvesting at given times to then return to the exponential growth phase.

### Arrays of helical tube photobioreactors in animal houses

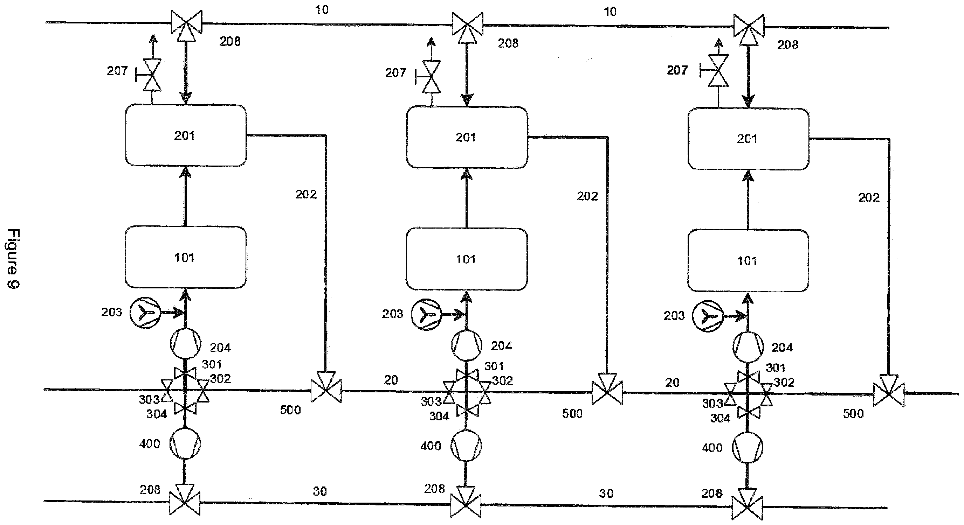

In one embodiment, an array of helical tube photobioreactor systems according to the invention is installed in an animal house by connecting a number of photobioreactors to supply pipelines via the valve-controlled inlets and outlets. This enables the operation of individual reactors either in series or in parallel. In order to have a maximum positive impact on the sequestration of gaseous and airborne components in the animal housing systems, in one embodiment, for commercial application in large livestock farms, a large number of the photobioreactors are installed in a single animal housing system. To control any number of such reactors, they are interconnected by serial and parallel flow streams. A non-limiting example is shown in Figure 9a. This allows continuous operation, maintenance, fertilization, harvesting, and replenishment for all reactors to be provided by only one set of central supply lines. This makes it possible to control all reactors connected to the supply lines by means of central logic circuitry from one operating area.

In a further embodiment, an array of photobioreactor systems according to the invention is installed in an animal house by connecting the photobioreactors to supply lines via the valve-controlled inlets and outlets in order to operate individual reactors individually with a culture solution by means of suitable flow control as well as to transfer the contents of one reactor to other reactors of the array. A non-limiting example is shown in Figure 9b. Such an array is used to perform multiplex studies of culture strains and cultivation conditions (e.g., different nutrient conditions) to optimize PMO cultivation and biomass production.

In a preferred embodiment, using the flow circuit shown in Figure 9b, different pre-cultures of microorganisms are first grown in individual bioreactors and mixed together after achieving an optimal cell density. This results in mixed multispecies cultures whose overall metabolism is determined by the mutual influence of individual species. It is known to the skilled person that mixed multispecies cultures can be used, for example, to produce variations in the composition of biomass and metabolites that are not accessible with pure cultures. Therefore, this embodiment makes it possible to produce value-added products with a high degree of automation, which has not been possible with conventional methods.

In another embodiment, the photobioreactor system according to the invention is used to culture a mixed community including PMO and higher organisms of the kingdom eukarya, such as free or submersed floating aquatic plants, saltwater or freshwater fish, or crustaceans. In this application, the photobioreactor system of the invention is configured with larger tube diameters, typically ranging from 0.1 to 0.5 m. In addition to the higher organisms, PMO are present in the culture medium to ensure a high oxygen concentration.

As used herein, the term "comprising"/"comprises" expressly includes the terms "consisting essentially ofʺ/"consists essentially of" and "consisting of'/"consists of", *i.e.,* all of said terms are interchangeable with each other herein.

Further, as used herein, the term "about" preferably represents a modifier of the specified value of ± 10%, more preferably ± 10%, ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention provides systems for the cultivation of photosynthetic microorganisms, that can be operated in conventional animal houses in order to use the emissions generated therein for the production of e.g. value-added compounds.

It is well known that various PMO, such as (micro)algae or cyanobacteria, can produce a variety of useful value-added products. For example, algal biomass has a huge market potential for products such as biofuels, hydrogen for energy storage and production, or high-value products for pharmaceuticals, cosmetics, food additives, biochemicals, and animal feed. To manufacture such products, various bioreactors have been developed as cultivation systems for PMO. However, of the multitude of bioreactors described to date as cultivation systems for PMO, no specific apparatus is known that allows the high production potential of PMO to be physically and functionally coupled with the emissions (gaseous and airborne components as well as metabolic waste heat) within a conventional animal house.

The present invention provides a system for the cultivation of PMO such as, but not limited to, *Cyanophyceae* (blue and green algae), *Chlorophyceae* (green algae), *Bacillariophyceae* (diatoms) and *Chrysophyceae* (golden algae), *Isochrysis* (golden brown flagellate *Prymnesiophyceae), Chaetoceros* (diatom), *Chlorella* (unicellular green algae - freshwater), *Arthrospira (Spirulina -* cyanobacteria (blue-green algae)), Diatoms *(Bacillariophyta)* or *Dunaliella* (unicellular green algae - saltwater) as well as bacterial and algal strains derived therefrom, in conventional animal houses in order to be able to harness the emissions produced therein by the animals for the production of valuable products in the sense of sustainability. Specifically, the present invention developed a system that takes into account a number of different features and incorporates them into a single, optimal cultivation system.

More specifically, to address a key aspect of the underlying technical problem, the present invention provides a system that employs a helically coiled tubular construction as a cultivation vessel such that the lower diameter of the helix is smaller than the upper diameter. This creates an overall conical shape that has a minimal footprint in the lower portion where animals are typically kept in an animal house. The conical tubular helix can be attached to the ceiling of the animal house by a tension-based flexible stabilizing framework. This load-bearing structure is not only easy to integrate into existing animal houses, but it is also highly resistant to prevent mechanical damage to the reactor and its internal operating elements (light fixtures, pumps, valves) due to physical contact with the animals.

The operation of the helical tube reactor according to the present invention works on the principle of an airlift reactor. For this purpose, at the bottom of the helical tube there is a gas inlet into which gas (e.g. ambient air from the animal house) can be blown through a fine particle filter with the aid of a pump. Due to its buoyancy, the injected gas causes a constant flow in the tube helix, which transports the contained nutrient medium and microorganisms suspended therein from the bottom to the top in the reactor tube. At the upper end, the liquid flow from the reactor tubing is directed into a reservoir, which serves as an equalizing vessel for the purpose of gas and pressure equalization and allows exchange with the outside air via a fine particle filter for protection against contamination. From this equalizing vessel, a vertical hose connection (vertical return tube) inside the helix structure leads the culture liquid downward from where it is again transported by the injected gas into the ascending helix. This ensures constant circulation of the culture medium and microorganisms contained therein. To achieve optimal growth of the PMO, a luminous body is integrated inside the helix, which supplies both the helical tube construction and the return tube in the center of the construction with light energy of suitable wavelength.

Due to its tailor-made design, the photobioreactor according to the invention can not only be optimally integrated into conventional animal houses, since its minimal footprint allows almost the entire floor space to continue to be used for the animals living in the house (pigs, cattle, sheep, poultry, etc.) and since its flexible suspension ensures a high degree of robustness to prevent mechanical damage by the animals. In addition, the helical structure of the photobioreactor provides a large contact area to absorb the waste heat in the lower part of the animal house, and by pumping in exhaust air from the lower part of the animal house, rapid mixing of the liquid culture phase is achieved. This creates the ideal growth temperatures and CO₂ concentrations for the cultivation of PMO.

To address other aspects of the underlying technical problem, the present invention also allows the photobioreactor to be easily scalable in its individual size and culture volume by changing the diameter and number of turns of the helix. Also, the photobioreactor according to the present invention can be easily integrated into series and parallel circuits, so that two or more reactors can be connected together to form an array, thereby optimizing the cultivation of PMO and the processing of biomass in terms of efficiency.

The PMO to be cultured in the system described herein may be any known photosynthetic microorganism. Such photosynthetic microorganisms may be any known species of oxygen releasing organisms and algae that may be cultured in the system described herein. These include, but are not limited to, the following species: *Nannochloropsis* sp., *Nannochloropsis salina, Nannochloris* sp., *Chlorella* salina, *Dunaliella tertiolecta, Dunaliella salina, Isochrysis sp., Isochrysis galbana, Tetraselmis suecica, Tetraselmis chuii, Phaeodactylum tricornutum, Coccomyxa sp..* The various microorganisms may be grown singly or as mixed species cultures, containing at least one type of PMO. Also, mixed communities with endosymbiotic members, e.g., bacterial endocytobionts, endosymbiotic ciliates, or microbial eukaryote with a combination of purple bacteria and green algae as endosymbionts can be cultured in the helical tube bioreactor of the invention.

The figures show:
Figure 1:
   Schematic three-dimensional representation of the helical tube photobioreactor according to the invention.
Figure 2:
   Two-dimensional cut in x/z or y/z direction of a cultivation system.
Figure 3:
   Possible shapes of the stiffening rings (106, in Figure 1).
Figure 4:
   Possible forms of vertical side braces.
Figure 5:
   Flow diagram of the helical tube reactor.
Figure 6:
   Growth curves of *Chlorella vulgaris* micro-algae.
Figure 7:
   Cultivation of *Chlorella* micro-algae in a mixed tube photobioreactor.
Figure 8:
   Growth of *Chlorella vulgaris* micro-algae in the presence of rotifers as predators.
Figure 9:
   Serial and parallel operation of multiple helical tube reactors.
Figure 10:
   Growth curves of a continuous culture of *Spirulina* sp. *(Athrospira platensis)* microalgae.

The present invention will be further illustrated by the following examples without being limited thereto.

### Examples

In order to understand the invention and to see how it may be carried out in practice, embodiments will now be described which serve as non-limiting examples. The following drawings are included to illustrate the photobioreactor system according to the invention.

**Figure 1** is a schematic three-dimensional representation of a cultivation system according to one embodiment of the invention. The three-dimensionality is shown by means of the coordinate system with x = length, y = depth and z = height. The system consists of a tubing that is helically wound (101) so that the lower diameter (102) is smaller than the upper diameter (103). This creates an overall conical shape, wherein the number of helical turns is determined by the height of the coiled tubing (104). The conical tubular construct is attached to the ceiling by a tension-based flexible stabilizing framework (105-107). Specifically, an array of stiffening rings at the upper edge of the helix (106) is attached to the ceiling with several flexible brackets (105) and serves as a holder for vertical side braces (107) to which the helical tube is attached. The array of stiffening rings contains at least one ring (106a) as holder for the vertical side braces (107) but it may also contain additional rings as carriers for the light source (106b) and/or a global scaffold ring (106c) that serves for the attachment of 106a, 106b as well as the carrier for the reservoir (201), the vertical return tube (202) and aeration pumps (203). Additional stiffening rings may be placed in the middle and lower sections of the coiled tubing to further stabilize the conical shape (not shown here). In order to operate the helical tube reactor according to the principle of an airlift reactor, there is a gas inlet (108) at the lower end of the tube helix, into which gas (e.g. ambient air) is injected with the aid of an aeration pump (203). The gas stream blown into the gas inlet (108) causes a constant flow of the nutrient medium contained in the coiled tubing from bottom to top (109) due to its buoyancy. At the upper end of the coiled tubing (109), the liquid flow from the reactor tubing is directed into a reservoir (201, see also Figure 2), which serves as an equalizing vessel for gas and pressure equilibration. From this equalizing vessel, a vertical return tube (202) inside the helical structure carries the culture fluid downward, from where it is transported by the injected gas back into the rising helix (108), so that a continuous circulation of the culture medium is achieved. In addition, a circulation pump (204) is integrated below, inside or above the conical pipe helix, with which the flow rates of the culture medium can be controlled independently of the pressure and buoyancy of the gas from the aeration compressor. The circulation pump (204) is not shown in this figure for clarity, but is shown in Figure 2. For the addition or removal of culture medium, the tubular reactor chamber is equipped with valves for the inlet (205) and outlet (206). Furthermore, a lighting fixture (110) is integrated inside the helix to provide light energy to both the helical tube structure and the return tube in the center of the structure. For clarity, the reservoir (201), the vertical return pipe (202), aeration (203) and circulation (204) pumps and the lighting fixture (110) are shown in a different geometric view in Figure 2.

**Figure 2** shows a two-dimensional cut in x/z or y/z direction of a cultivation system according to one embodiment of the invention. Cross sections of the individual turns of the spirally wound tubing (101) are indicated by the gray circles. The tubular construct is attached to the ceiling by a tension-based flexible stabilizing framework consisting of an array of stiffening rings (106) at the upper edge of the helix that is attached to the ceiling with several flexible brackets (105) and serves as a holder for vertical side braces (107) to which the helical tube is attached. Additional stiffening rings may be placed in the middle and lower sections of the coiled tubing to further stabilize the conical shape (not shown here). To enable operation according to the principle of an airlift reactor, there is a gas inlet (108) at the lower end of the tube helix, into which gas (e.g. ambient air) is injected with the aid of an aeration pump (203) to establish a constant flow of the nutrient medium inside the tube helix from the bottom (108) to the top (109).

At the upper end (109), the liquid flow from the reactor tubing is directed into a reservoir (201) that serves as an equalizing vessel for gas and pressure equilibration. From this equalizing vessel, a vertical return tube (202) in the center of the helix construct carries the culture fluid downward, from where it is transported by the injected gas back into the rising helix (108), so that a continuous circulation of the culture medium is achieved. In addition, a circulation pump (204) is integrated below the conical pipe helix, with which the flow rates of the culture medium can be controlled independently of the pressure and buoyancy of the gas from the aeration pump (203). The position of the circulation pump (204) can be integrated below, inside or above the conical tube coil, but is selected below here for reasons of clarity. A lighting fixture (110) is integrated inside the helix to provide light energy to both the helical tube structure and the return tube in the center of the structure.

**Figure 3** shows possible shapes of the stiffening rings (106, in Figure 1), shown schematically as a top view from the Z direction. The shape of the stiffening ring is decisive for the external shape of the reactor and can be, for example, circular (a), oval (b), or have the resulting shape of a cut circle extended by parallel sides of any length (c). Any polygonal shape can be realized by appropriately shaped stiffening rings (d).

**Figure 4** shows possible forms of vertical side braces (107, in Figure 1). The vertical side braces connect the upper and lower stiffening rings of the spiral tube, and they serve as support elements for the reactor tubing. The vertical side braces consist of rigid (solid lines) or flexible (dashed lines) elements. They are connected to the stiffening rings either directly and inflexibly (Figure 4a) or via flexible, non-rigid elements (Figure 4b). The vertical side braces may consist entirely of flexible elements (Figure 4c), of a number of alternating rigid and flexible non-rigid spacer elements (Figure 4d) or as short rigid or flexible spacer elements which have a clip at their upper and lower ends as a bracket, thus connecting tube segments located one above the other (Figure 4e). Furthermore the vertical side brace can also hold the helical tube in place externally with respect to the central helical axis (Figure 4f). The angle α is the angle of inclination of the truncated cone between the vertical side braces and the cone's central axis

**Figure 5** shows the flow diagram of the helical tube bioreactor. The figure shows the flow diagram of the nutrient solution in one embodiment of the photobioreactor according to the invention. The nutrient medium is circulated by the gas stream blown into the inlet of the helically wound reactor tube by means of an aeration compressor (203). Due to the buoyancy of the gas from the aeration compressor, the nutrient medium is passed through the helically wound reactor tube (101) into the equalizing reservoir (201) and from there through the vertical return pipe (202) back into the helically wound reactor tube (101) and until it reaches the lower gas inlet (108 in Figure 1, not shown here). To control the flow rates of the nutrient medium independently of the pressure and buoyancy of the gas from the aeration compressor (203), one or more circulation pumps (204) are integrated into the system. The inlet (205) and outlet (206) valves connect the photobioreactor to central supply lines (see Figure 9), which allow the addition (via inlet 205) or removal (via outlet 206), respectively, of fluids without interrupting continuous operation. The gas outlet (207) allows excess gas to escape the reactor.

**Figure 6** shows growth curves of *Chlorella vulgaris* micro-algae, obtained from the cultivation of the micro-algae at 23°C, indoor air moved with a compressor, 16h light obtained from 6 lamps (Valoya, L28-AP673L), culture medium(1,2g/l KNO3 + 0,5ml/l, Wuxal NPK (8/8/6) fertilizer), in either the helical tube bioreactor (squares) or the conventional cultivation in a plastic bag (triangles).

**Figure 7** shows the cultivation of *Chlorella vulgaris* micro-algae in the helical tube photobioreactor according to the invention. Section a) of the figure shows a photograph of the reactor, in which the helically coiled cultivation tube is composed of two tube sections made of PVC (top, black arrow) and silicone (bottom, white arrow) connected with each other by aid of flanged sleeves (black element in between the dashed circles). At the time of the photograph, the airlift pump was turned off to make the difference in bioadhesion between PVC and silicone more apparent. Note that no significant biofilm adhesion has occurred in the upper silicone part (see magnified inset b), whereas a strong deposition of biofilm can be observed in the lower PVC part (inset c).

**Figure 8** shows the model production of a multispecies culture of micro-algae and Ciliate, in which a predator-prey relationship is exploited for biomass variation in a helical tube photobioreactor. In a), the growth curve of *Chlorella vulgaris* is shown, exhibiting exponential growth until the population of micro-algae reaches a maximum, which is the threshold to allow rapid growth of the Ciliate. This results in the decrease of micro-algae and the simultaneous growth of Ciliate. The micrographs show the pure *Chlorella* culture (b), t = 1d) and the increase in rotifers at t = 18d (c) and t = 20d (d). Scale bars are 0,1 mm (b) and 1 mm (c, d).

**Figure 9** shows the flow diagram for serial and parallel operation of multiple helical tube bioreactors. In order to be able to use the photobioreactor according to the invention in an economically reasonable way in large animal husbandry facilities, several plants are installed in parallel so that they can be filled or emptied from a central location. The flow diagram shown in Figure 9 is an example of three photobioreactors (101) connected in series, which are interconnected via line 10 for the supply of nutrient medium and line 30 as the removal line. The elements 201 (equalizing reservoir), 202 (return pipe), 203 (aeration compressor) and 204 (circulation pump) of the individual photobioreactors described in Figures 1 and 2 are also shown. All or individual reactors can be filled via line 10, e.g. in order to initially fill the reactors with nutrient medium or to make up for volumes removed. Through line 30, cultivation media can be drained from individual or all reactors simultaneously, e.g. in the course of harvesting. Because of the controllable line valves 207 in lines 10 and 30, these operations can be performed on individual reactors or simultaneously on defined groups or all reactors. In addition, a circulation pump 400 is integrated between reactor 101 and line 30 so that liquid volumes from 30 can also be transported into reactor 101 against gravity by adjusting 3-way valve 207 accordingly. Line 20, which is connected to the individual reactors via 3-way valve 500 and valves 301, 302, 303 and 304 is used to enable more advanced filling and transfer operations. Line 20, which is connected to the individual reactors via 3-way valve 500 and valves 301, 302, 303 and 304 is used to enable more advanced filling and transfer operations. Thus, this flow circuit enables, for example, to grow different PMO cultures in individual reactors, to harvest them individually or to realize the transfer of PMO cultures between any reactors. It is also possible to flush individual or multiple reactors. Since lines 10 and 30 can be centrally loaded and unloaded and all valves and pumps can be remotely controlled, this arrangement of reactors allows for wide variability of cultivation processes.

**Figure 10** shows an example in which a mixed culture of *Spirulina* sp. microalgae *(Athrospira platensis)* and diatoms in a culture medium (water containing 203 mg (N)/l; 35 mg (S)/l; 4,3 mg (Fe)/I; 344 mg (K)/l; 51 mg (P)/l; 17 mg (Mg)/I; 5g (NaCl)/l) is continuously cultured in the helical tube photobioreactor system according to the invention. The Reactor was operated for continuous cultivation over the course of 44 day, including three harvests at day 11, 20, and 33. The cultivation occurred at an average temperature of 24°C, the medium was ventilated and agitated with an air compressor and illuminated for 16h/d by 6 lamps (Valoya, L28-AP673L). The growth of *Spirulina* was monitored by determination of the concentration of the microalgae in grams (dry weight) per liter culture medium. Figure 10 illustrates that there is continuous rapid growth of microalgae in the helical tube photobioreactor, which can be reset to a low growth value by harvesting at appropriate times to then return to the exponential growth phase.

## Claims

1. A photobioreactor system for the cultivation of photosynthetic microorganisms, comprising
(a) a cultivation vessel comprising at least one tubular element (101) extending helically around a center axis, wherein the diameter (102; 103) of the helix thus formed continuously increases as the tubular element extends along the center axis,
(b) at least one gas inlet (108) at a first end of the tubular element (101) configured to provide a gas to the tubular element (101),
(c) an equalizing reservoir (201) in fluid connection with a second end (109) of the tubular element (101) configured to provide pressure equalization in the cultivation vessel,
(d) at least one fluid return element (202) fluidly connecting the equalizing reservoir (201) and the first end of the tubular element (101), wherein the tubular element (101) at least partially surrounds the fluid return element (202), and
(e) at least one lighting fixture (110) configured to supply both the tubular element (101) and the fluid return element (202) with light energy of a suitable wavelength, wherein the tubular element (101) at least partially surrounds the lighting fixture (110).

2. . The photobioreactor system of claim 1, further comprising a stabilizing framework (105, 106, 107) supporting the cultivation vessel, wherein the stabilizing framework is configured to mount the photobioreactor system in a hanging manner to a support.

3. . The photobioreactor system of claim 2, wherein the stabilizing framework (105, 106, 107) comprises an array of stiffening rings at the upper edge of the helix (106) that is attachable to the support with flexible brackets (105) and serves as a holder for vertical side braces (107) to which the tubular elements (101) are attached.

4. . The photobioreactor system of claim 3, wherein the array of stiffening rings comprises
(i) at least one ring (106a) as holder for the vertical side braces (107), and/or
(ii) additional rings as carriers for the light fixtures (106b) and/or a global scaffold ring (106c) that serves for the attachment of 106a, 106b as well as the carrier for the equalization reservoir (201), and the fluid return elements (202).

5. . The photobioreactor system of any one of claims 1 to 4, further comprising
(i) an aeration pump (203) configured to provide gas into said gas inlet (108),
(ii) inlet (205) and outlet valves (206) for the addition or removal of culture medium,
(iii) one or more sensor units for measuring optical density, pH value, and/or chemical composition of the culture fluid,
(iv) one or more gas outlet valves (207) to allow excess gas to escape the system, and/or
(v) a secondary air inlet attached at the lowest point of the equalizing reservoir (201 )/vertical return tube (202) to provide air bubble-induced turbulence to improve mixing and prevent sedimentation of biomass in the equalizing reservoir (201 )/vertical return tube (202).

6. . The photobioreactor system of any one of claims 1 to 5, wherein the cultivation vessel comprises one or more additional tubular elements (101) extending helically around a center axis, wherein the diameter (102; 103) of the helix thus formed continuously increases as the tubular element extends along the center axis.

7. . The photobioreactor system of claim 6, wherein the tubular elements (101) are arranged (i) as a helix in which the ascending helices are alternately stacked, or (ii) in multiple layers.

8. . The photobioreactor system of any one of claims 1 to 7, further comprising one or more additional lighting fixtures (110).

9. . The photobioreactor system of any one of claims 1 to 8, wherein the lighting fixtures are disposed in between tubular element helices and/or in between tubular element layers.

10. . The photobioreactor system of any one of claims 1 to 9, wherein the equalizing reservoir (201) and the at least one fluid return elements (202) are combined and are located in the center of the bioreactor system, thereby forming a centrally arranged equalization reservoir that also serves as a fluid return element.

11. . The photobioreactor system of any one of claims 1 to 10, wherein
(i) the lower, smallest helix diameter (102) is from about 0.10 m to about 5.00 m,
(ii) the upper, largest helix diameter (103) is from about 0.20 m to about 15.00 m,
(iii) the height (104) of the helix is from about 0.30 m to about 8.00 m, and/or
(iv) the number of helix turns is from about 10 to about 150 helix turns.

12. . The photobioreactor system of any one of claims 1 to 11, wherein
(i) the outer diameter of a tubing forming the tubular element (101) is from about 0.01 m to about 0.5 m, and/or
(ii) the wall thickness of a tubing forming the tubular element (101) is between about 0.01 mm and about 25 mm.

13. . The photobioreactor system of any one of claims 1 to 12, wherein the tubular element (101) and/or the fluid return element (202) and/or the equalizing reservoir (201) are made of a material, selected from the group consisting of silicone rubber, silicone rubber composites, PVC (polyvinylchloride), polyethylene (PE), HDPE (high density polyethylene), PP (polypropylene), PET (polyethylene terephalate), acrylate (plexiglas), fiberglass (glass-reinforced plastic (GRP)) polyester, fiberglass (GRP), glass, and combinations thereof.

14. . An array comprising two or more individual photobioreactor systems of any one of claims 1 to 13, wherein the individual photobioreactor systems are connected and integrated into a serial or parallel circuit.

15. . Use of one or more photobioreactor systems of any one of claims 1 to 13 or an array of claim 14 for converting waste gases produced in an animal house to biomass.
